# EUROPEAN PATENT APPLICATION

(11) **EP 4 821 526 A1**
(43) Date of publication of application: **14.10.2026**
(21) Application number: 24900812.9
(22) Date of filing: 04.10.2024
(51) Int. Cl.: A61M 15/00

(54) **INHALER**

(30) Priority: 07.12.2023 KR 20230177004
(71) Applicant: KT&G Corporation, Daedeok-gu Daejeon 34337 (KR)
(72) Inventor: KIM, Moonwon, Daejeon 34187 (KR); SEO, Man Seok, Seoul 05387 (KR); JUNG, Yong Mi, Sejong 30153 (KR); SHIN, Jun Won, Sejong 30098 (KR)
(74) Representative: Ter Meer und Partner mbB
(86) International application number: PCT/KR2024/015063
(87) International publication number: WO 2025/121624

(57) **Abstract**

An inhaler according to an embodiment comprises: a housing which comprises a first surface, a second surface facing the first surface, and a side surface connecting the first and second surfaces, and which is provided with an elongated cavity, extending from the first surface toward the second surface, into which an aerosol-generating item including a capsule is inserted; a first needle disposed perpendicular to the second surface and extending in the longitudinal direction from the second surface toward the first surface; and a second needle adjacent to the side surface and disposed in the transverse direction perpendicular to the longitudinal direction, wherein the first needle crushes the capsule in the longitudinal direction, and the second needle can penetrate the side surface and move forward or backward so as to crush the capsule in the transverse direction.

## Description

### Technical Field

Methods and apparatuses consistent with embodiments relate to an inhaler.

### Background Art

A conventional dry powder inhaler is intended to transfer a single-dose, metered dose of a pharmacological agent into the body and has been used primarily to treat patients with asthma and chronic obstructive pulmonary disease (COPD). Finely dried powders of 5 µm to 10 µm or less are used for efficient absorption of the agent into the body. In addition, a determined amount of drug may be filled into a hard capsule for quantitative delivery.

The dry powder inhaler may consist of a capsule filled with dry powder and an inhalation device. For this purpose, technology for manufacturing dry powder and technology for designing a device that generates a vortex for delivering dry powder may be required.

The above description is information the inventor(s) acquired during the course of conceiving the present disclosure, or already possessed at the time, and is not necessarily art publicly known before the present application was filed.

### Disclosure of the Invention

### Technical Problem

Embodiments provide an inhaler that may be coupled to an inhalation stick storing a capsule filled with dry powder for transferring inhalation dry powder and that includes a separate perforation function for generating a vortex near an inhalation capsule, in addition to a perforation function for the inhalation capsule in the stick, thereby allowing direct perforation during a capsule perforation process for inhalation by a user without a need to manufacture a separate vortex-generating structure within the inhaler in a manufacturing process of an inhaler and thus preventing contamination near the capsule due to an external environment during storage of the stick.

The technical goals obtainable from the embodiments are not limited to the above-mentioned technical goals, and other unmentioned technical goals may be clearly understood from the following description by one of ordinary skill in the art to which the present disclosure pertains.

### Solution to Problem

According to an aspect of an embodiment, there is provided an inhaler including a housing, which includes a first surface, a second surface opposite to the first surface, and a side surface connecting the first surface to the second surface, wherein an elongated cavity is formed in the housing, the elongated cavity extending from the first surface toward the second surface and into which an aerosol-generating article comprising a capsule is inserted, a first needle disposed perpendicular to the second surface and extending in a longitudinal direction from the second surface toward the first surface, a second needle adjacent to the side surface and disposed in a transverse direction perpendicular to the longitudinal direction, wherein the first needle is configured to crush the capsule in the longitudinal direction, and wherein the second needle is configured to move forward or backward penetrating the side surface to crush the capsule in the transverse direction.

### Advantageous Effects of Invention

According to embodiments, an inhaler may be coupled to an inhalation stick storing a capsule filled with dry powder for transferring inhalation dry powder and may include a separate perforation function for generating a vortex near an inhalation capsule, in addition to a perforation function for the inhalation capsule in the stick, thereby allowing direct perforation during a capsule perforation process for inhalation by a user without a need to manufacture a separate vortex-generating structure within the inhaler in a manufacturing process of an inhaler and thus preventing contamination near the capsule due to an external environment during storage of the stick.

The effects of the manipulator according to an embodiment are not limited to the above-mentioned effects, and other unmentioned effects can be clearly understood from the following description by one of ordinary skill in the art to which the present disclosure pertains.

### Brief Description of Drawings

FIG. 1 is a diagram illustrating a first needle, a second needle, and an aerosol-generating article of an inhaler, according to an embodiment;
FIG. 2 is a diagram illustrating an inhaler into which an aerosol-generating article is inserted, according to an embodiment;
FIG. 3 is a diagram illustrating an internal perspective view of the inhaler into which the aerosol-generating article is inserted, according to an embodiment;
FIG. 4 is a diagram illustrating a longitudinal section of the inhaler into which the aerosol-generating article is inserted, according to an embodiment;
FIG. 5 is a diagram illustrating a cross section of the inhaler in which a second needle is in a retracted state, according to an embodiment;
FIG. 6 is a diagram illustrating a cross section of the inhaler in which a second needle is in an advanced state, according to an embodiment; and
FIG. 7 is a diagram illustrating a cross section of the inhaler including three first needles, according to an embodiment.

The accompanying drawings illustrate desired embodiments of the present disclosure and are provided together with the detailed description for better understanding of the technical idea of the present disclosure. Therefore, the present disclosure should not be construed as being limited to the embodiments set forth in the drawings.

### Best Mode for Carrying Out the Invention

Hereinafter, embodiments are described in detail with reference to the illustrative drawings. Regarding the reference numerals assigned to the components in the drawings, it should be noted that the same components are designated by the same reference numerals, wherever possible, even though they are shown in different drawings. Furthermore, in the description of the embodiments, detailed description of well-known related configurations or functions is omitted when it is deemed that the description will cause ambiguous interpretation of the embodiments.

In addition, in the description of the components of the embodiments, terms such as first, second, A, B, (a), (b), and the like may be used. These terms are used only for the purpose of discriminating one component from another component, and the nature, the sequences, the orders, or the like of the components are not limited by the terms. It is to be understood that if a component is described as being "connected," "coupled," or "joined" to another component, the former may be directly "connected," "coupled," or "joined" to the latter or "connected," "coupled," or "joined" to the latter via another component.

The same name may be used to describe components having a common function in different embodiments. Unless otherwise mentioned, the description of one embodiment may be applicable to another embodiment. Thus, duplicated description is omitted for conciseness.

The present disclosure relates to an inhaler 10 for transferring inhalation dry powder, and more specifically, to the inhaler 10 exclusive for an inhalation stick or an aerosol-generating article storing a capsule filled with dry powder.

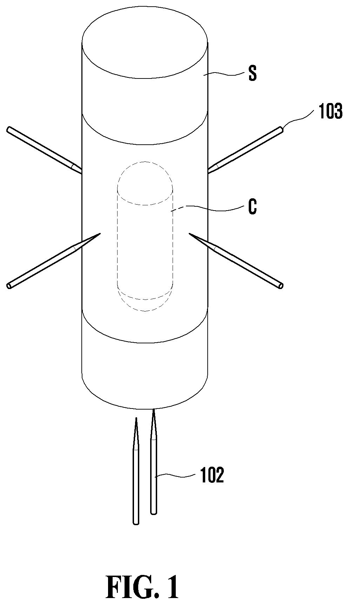

FIG. 1 is a diagram illustrating a first needle 102, a second needle 103, and an aerosol-generating article S of the inhaler 10, according to an embodiment.

Referring to FIG. 1, the inhaler 10 may perforate a capsule C included in the aerosol-generating article S. The inhaler 10 may provide a perforation function for inhaling a functional material contained within the capsule C and for generating a vortex. For example, the first needle 102 may perform perforation for inhaling the functional material, and the second needle 103 may perform perforation for generating a vortex.

A material such as paper, cellulose acetate tow, and the like, and a polymer material such as polylactide (PLA), polypropylene (PP), polyethylene (PE), and the like may be applied to the aerosol-generating article S. A flavor capsule may be applied to the aerosol-generating article S.

The capsule C may contain a functional material in a dry powder form. Dry powder suitable for inhalation through a structure of the inhaler 10 may be composed of a plurality of fine particles having a size less than or equal to 10 µm or a plurality of particles having an aerodynamic diameter less than or equal to 5 µm. Alternatively, some flavoring or cough suppressant particles of a size greater than or equal to 20 µm may be mixed into the dry powder. In addition, the dry powder may contain a small amount of a material that has a positive effect on the properties of the dry powder, such as other amino acids and magnesium stearate.

The dry powder that may be applied to an inside of the inhaler 10 may include nicotine. For example, the nicotine may be free nicotine, nicotine lactate, or other nicotine salts. In addition, the dry powder may be provided as other functional materials.

A small amount of sweetener such as menthol, saccharin, sucralose, stevia, and the like may be added to the functional material of the inhaler 10 as a particle for flavor or taste. The sweetener may be in a fine powder form. In this case, the sweetener may be added in an amount of less than 1 to 5 wt% in the nicotine dry powder. A size of the flavor particle may be less than or equal to 5 µm or greater than or equal to 20 µm on average. Alternatively, the size may be 50 µm to 100 µm.

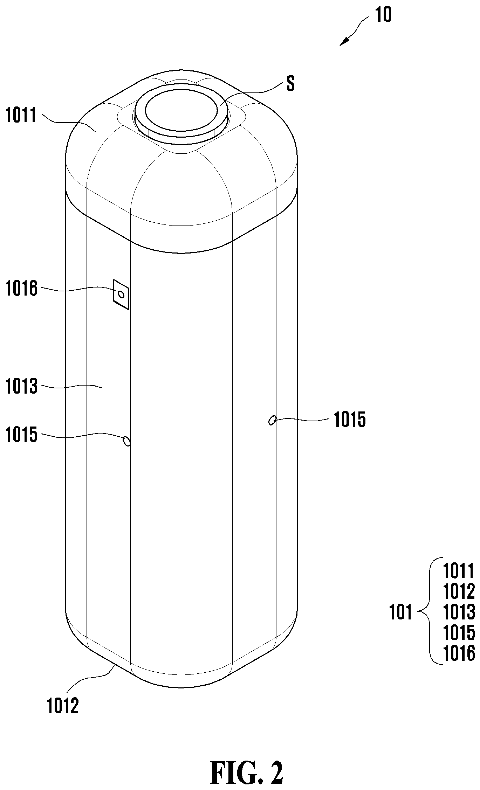

FIG. 2 is a diagram illustrating the inhaler 10 into which the aerosol-generating article S is inserted, according to an embodiment.

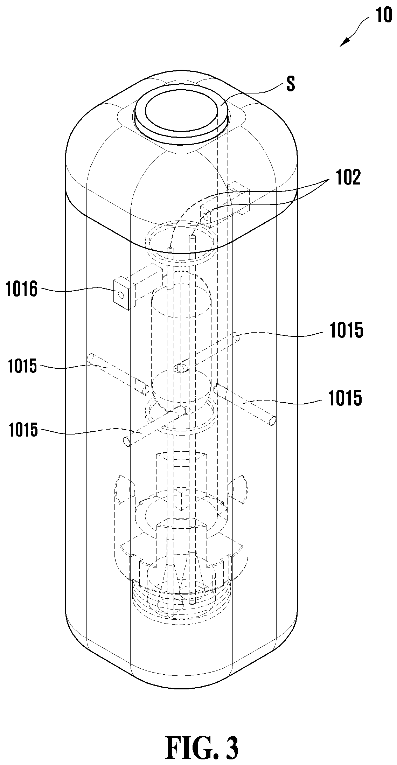

FIG. 3 is a diagram illustrating an internal perspective view of the inhaler 10 into which the aerosol-generating article S is inserted, according to an embodiment.

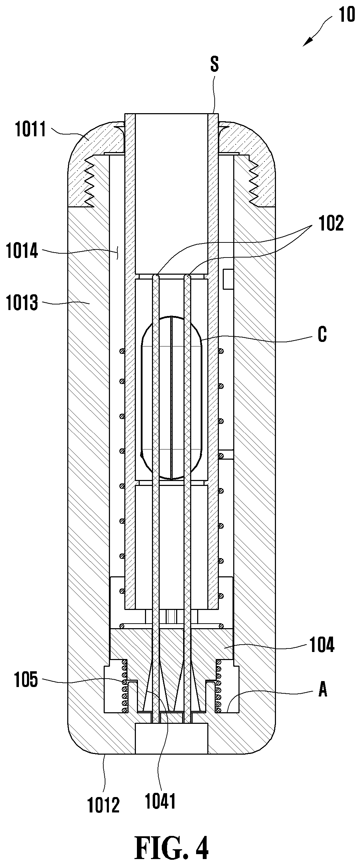

FIG. 4 is a diagram illustrating a longitudinal section of the inhaler 10 into which the aerosol-generating article S is inserted, according to an embodiment.

Referring to FIGS. 1 to 4, the inhaler 10 may include a housing 101, a first needle 102, and a second needle 103.

Referring to FIG. 2, the housing 101 may include a first surface 1011, a second surface 1012 opposite to the first surface 1011, and a side surface 1013 connecting the first surface 1011 to the second surface 1012. In addition, the first surface 1011 of the housing 101 may be open, and an elongated cavity 1014 extending from the first surface 1011 toward the second surface 1012 may be formed in the housing 101, as shown in FIG. 4. The aerosol-generating article S including the capsule C may be inserted into the elongated cavity 1014.

Referring to FIGS. 3 and 4, the first needle 102 may be disposed perpendicular to the second surface 1012. The first needle 102 may extend in a longitudinal direction from the second surface 1012 toward the first surface 1011. The first needle 102 may crush the capsule C in the longitudinal direction. The first needle 102 may be provided as a needle having a sharp shape. A needle size may range from 0.5 mm to 1.0 mm. The first needle 102 may be provided in one or more pieces.

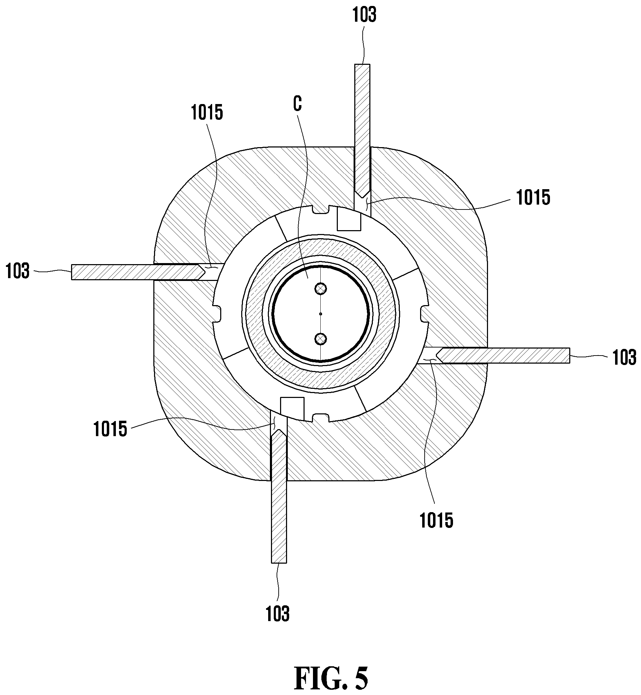

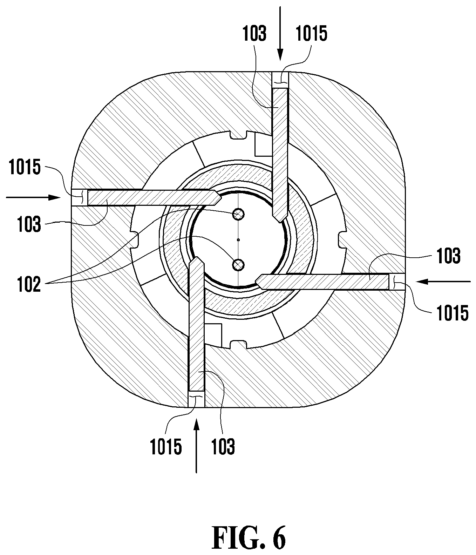

The second needle 103 may be adjacent to the side surface 1013 and be disposed in a transverse direction perpendicular to the longitudinal direction. The second needle 103 may operate to move forward or backward by penetrating the side surface 1013. By the forward or backward motion, the second needle 103 may crush the capsule C in the transverse direction. The second needle 103 may act, for example, in a tangential direction of the capsule C. The second needle 103 that may provide this perforation function may form a hole for generating a vortex in the capsule C. The second needle 103 may be provided as a needle having a sharp shape. A needle size may be greater than or equal to 0.5 mm. The second needle 103 may be one or two and may be provided in plurality to be suitable for generating a vortex. The second needle 103 described above is described in more detail below with reference to FIGS. 5 and 6.

Referring to FIGS. 2 and 3, the housing 101 may further include a needle guide 1015. The needle guide 1015 may be formed as a hole that penetrates the side surface 1013 in a transverse direction. The second needle 103 may be accommodated in the needle guide 1015. The needle guide 1015 may be provided in plurality. Each of the needle guides 1015 may be disposed spaced apart from each other in a circumferential direction on the side surface 1013. The needle guides 1015 may guide the second needle 103 to move forward or backward toward the capsule C.

Referring to FIGS. 2 and 3, the housing 101 may further include an air inlet 1016. The air inlet 1016 may extend from the side surface 1013 to the elongated cavity 1014. The air inlet 1016 may communicate the elongated cavity 1014 with an outside.

Referring to FIGS. 3 and 4, the inhaler 10 may further include a sliding portion 104. The sliding portion 104 may be joined to one end of the aerosol-generating article S. The sliding portion 104 may make a sliding movement in the elongated cavity 1014. When the sliding portion 104 makes a sliding movement in a direction from the first surface 1011 toward the second surface 1012, the first needle 102 may penetrate the sliding portion 104. That is, when the sliding portion 104 slides in the direction from the first surface 1011 toward the second surface 1012, the first needle 102 may crush the capsule C.

Specifically, a plurality of needle holes 1041 penetrating the sliding portion in the longitudinal direction may be formed in the sliding portion 104. A number of the needle holes 1041 may correspond to a number of the first needle(s) 102. The needle holes 1041 may be formed in a shape in which a diameter of an opening adjacent to the second surface 1012 is greater than a diameter of an opening adjacent to the first surface 1011, as shown in FIG. 4. For example, the needle holes 1041 may be formed in a trumpet shape. The shape of the needle holes 1041 may allow the first needle 102 to smoothly reach the capsule C by penetrating the needle holes 1041.

Referring to FIGS. 3 and 4, the inhaler 10 may further include a spring 105. The spring 105 may have one end coupled to the sliding portion 104 and an other end fixed to a bottom surface A of the elongated cavity 1014. The spring 105 may contract when the sliding portion 104 is pressurized and moves toward the second surface 1012. Accordingly, the first needle 102 may penetrate the sliding portion 104 and the aerosol-generating article S to perforate the capsule C. For example, the first needle 102 consisting of two needles may be arranged at a distance less than a diameter of the spring 105 and may be arranged to be surrounded by the spring 105.

In addition, the spring 105 may support the sliding portion 104 to be positioned spaced apart from the first needle 102 and toward the first surface 1011 when the sliding portion 104 is not pressurized.

Hereinafter, an operation of the second needle 103 is described in detail with reference to FIGS. 5 and 6.

FIG. 5 is a diagram illustrating a cross section of the inhaler 10 in which the second needle 103 is in a retracted state, according to an embodiment.

FIG. 6 is a diagram illustrating a cross section of the inhaler 10 in which the second needle 103 is in an advanced state, according to an embodiment.

Referring to FIGS. 5 and 6, the plurality of needle guides 1015 described above may each be disposed eccentrically from a central axis of the housing 101. For example, the needle guides 1015 may be disposed in the tangential direction of the capsule C. The second needle 103 may pass through the needle guides 1015 and perforate the capsule C at a position eccentric from the central axis.

A forward or backward motion of the second needle 103 may be performed in conjunction with a perforation by the first needle 102. For example, the second needle 103 may operate to move forward or backward in conjunction with a position of the sliding portion 104.

To this end, the inhaler 10 may further include a detection portion (not shown) and a driving portion (not shown).

The detection portion may be installed on the bottom surface A of the elongated cavity 1014 and may protrude toward the first surface 1011.

The driving portion may operate the second needle 103 to move forward or backward according to a signal detected by the detection portion.

The detection portion described above may signal the driving portion so that the second needle 103 may move forward or backward in conjunction with an operation of the sliding portion 104.

For example, the detection portion may signal the driving portion so that the second needle 103 may advance toward the capsule C when the sliding portion 104 moves in the direction from the first surface 1011 toward the second surface 1012. By the signal from the detection portion, the driving portion may move the second needle 103 from a position shown in FIG. 5 in a direction of the arrows shown in FIG. 6. Accordingly, the second needle 103 may form an eccentric perforation in the capsule C as shown in FIG. 6. The perforation formed by the second needle 103 may form a vortex toward an interior of the capsule C when an inhaling force is applied to the inhaler 10 by a user.

In addition, the detection portion may signal so that the second needle 103 may move backward from the capsule C toward an outer side of the housing 101 when the sliding portion 104 moves in a direction from the second surface 1012 toward the first surface 1011. Accordingly, the driving portion may return the second needle 103 from a position shown in FIG. 6 to the position shown in FIG. 5.

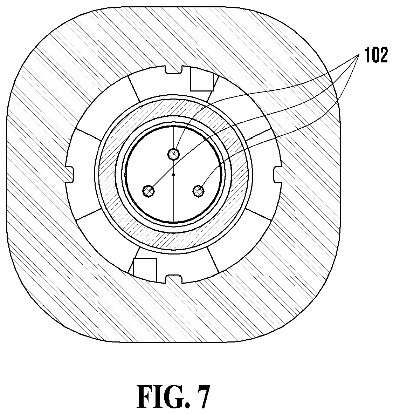

FIG. 7 is a diagram illustrating a cross section of the inhaler 10 including three first needles 102, according to an embodiment.

The first needle 102 shown in FIGS. 1 to 6 is exemplarily illustrated as consisting of two needles. However, as shown in FIG. 7, the first needle 102 may consist of three or more needles for smooth transfer and inhalation of the functional material.

As described above, the inhaler 10 may be coupled to an aerosol-generating article S storing a capsule C filled with dry powder for transferring inhalation dry powder and may provide a transverse perforation function for generating a vortex near the capsule C, in addition to a longitudinal perforation function for the capsule C, thereby allowing direct perforation during the capsule C perforation process for inhalation by a user without a need to manufacture a separate vortex-generating structure within the inhaler 10 when manufacturing the inhaler 10 and thus preventing contamination near the capsule C due to an external environment during storage of the aerosol-generating article S.

The inhaler 10 may include the housing 101 including the first surface 1011, the second surface 1012 opposite to the first surface 1011, and the side surface 1013 connecting the first surface 1011 to the second surface 1012, wherein the first surface is open, and wherein the elongated cavity 1014, into which the aerosol-generating article S including the capsule C is inserted, is formed in the housing 101, the first needle 102 disposed perpendicular to the second surface 1012 and extending in the longitudinal direction from the second surface 1012 toward the first surface 1011, the second needle 103 adjacent to the side surface 1013 and disposed in the transverse direction perpendicular to the longitudinal direction, wherein the first needle 102 may be configured to crush the capsule C in the longitudinal direction, and wherein the second needle 103 may be configured to move forward or backward penetrating through the side surface 1013 to crush the capsule C in the transverse direction.

The housing 101 may further include the needle guide 1015 formed as a hole penetrating the side surface 1013 in the transverse direction and capable of accommodating the second needle 103, and the needle guide 1015 may guide the second needle 103 so that the second needle 103 may move toward the capsule C.

The needle guide 1015 may be disposed eccentrically from the central axis of the housing 101.

The needle guides 1015 may be disposed in the tangential direction of the capsule C.

The housing 101 may further include the air inlet 1016 extending from the side surface 1013 to the elongated cavity 1014, and the air inlet 1016 may communicate the elongated cavity 1014 with the outside.

The inhaler 10 may further include the sliding portion 104 to which one end of the aerosol-generating article S may be coupled and which may make a sliding movement in the elongated cavity 1014, wherein the first needle 102 may penetrate the sliding portion 104 when the sliding portion 104 makes a sliding movement in a direction from the first surface 1011 toward the second surface 1012.

The inhaler 10 may further include a detection portion installed on the bottom surface A of the elongated cavity 1014 and protruding toward the first surface 1011 and the driving portion configured to operate the second needle 103 according to a signal detected by the detection portion, wherein the detection portion may signal the driving portion so that the second needle 103 may move forward or backward in conjunction with the operation of the sliding portion 104.

The detection portion may signal so that the second needle 103 may move forward when the sliding portion 104 moves in a direction from the first surface 1011 toward the second surface 1012 and may signal so that the second needle 103 may move backward when the sliding portion 104 moves in the longitudinal direction.

The plurality of needle holes 1041 penetrating the sliding portion 104 in the longitudinal direction may be formed in the sliding portion 104, and the needle holes 1041 may have the diameter of an opening adjacent to the second surface 1012 greater than the diameter of an opening adjacent to the first surface 1011.

The inhaler 10 may further include the spring 105 having one end coupled to the sliding portion 104 and the other end coupled to the bottom surface A of the elongated cavity 1014, wherein the spring 105 may contract when the sliding portion 104 is pressurized and moves toward the second surface 1012, and wherein the spring 105 may support the sliding portion 104 to be positioned spaced apart from the first needle 102 and toward the first surface 1011 when the sliding portion 104 is not pressurized.

The capsule C may contain the functional material in a powder form.

While the embodiments of the present disclosure have been described above with reference to specific components, and limited embodiments and drawings, the above descriptions are merely for better understanding of the present disclosure, and it will be apparent to one of ordinary skill in the art that various changes in form and details may be made in these embodiments without departing from the spirit and scope of the claims and their equivalents. For example, suitable results may be achieved if the described techniques are performed in a different order, and/or if components in a described architecture, device, and the like are combined in a different manner and/or replaced or supplemented by other components or their equivalents. Accordingly, the scope of the present disclosure is defined not by the detailed description, but by the claims and their equivalents, and all variations within the scope of the claims and their equivalents are to be construed as being included in the disclosure.

## Claims

1. An inhaler comprising:
a housing comprising a first surface, a second surface opposite to the first surface, and a side surface connecting the first surface to the second surface, wherein an elongated cavity is formed in the housing, the elongated cavity extending from the first surface toward the second surface and into which an aerosol-generating article comprising a capsule is inserted;
a first needle disposed perpendicular to the second surface and extending in a longitudinal direction from the second surface toward the first surface; and
a second needle adjacent to the side surface and disposed in a transverse direction perpendicular to the longitudinal direction,
wherein the first needle is configured to crush the capsule in the longitudinal direction, and
wherein the second needle is configured to move forward or backward penetrating through the side surface to crush the capsule in the transverse direction.

2. The inhaler of claim 1, wherein
the housing further comprises a needle guide formed as a hole penetrating the side surface in the transverse direction and configured to accommodate the second needle, and
the needle guide is configured to guide the second needle to move toward the capsule.

3. The inhaler of claim 2, wherein the needle guide is disposed eccentrically from a central axis of the housing.

4. The inhaler of claim 3, wherein the needle guide is disposed in a tangential direction of the capsule.

5. The inhaler of claim 1, wherein
the housing further comprises an air inlet extending from the side surface to the elongated cavity, and
the air inlet is configured to communicate the elongated cavity with an outside.

6. The inhaler of claim 1, further comprising:
a sliding portion to which one end of the aerosol-generating article is coupled and which is configured to make a sliding movement in the elongated cavity,
wherein the first needle is configured to penetrate the sliding portion when the sliding portion makes a sliding movement in a direction from the first surface to the second surface.

7. The inhaler of claim 6, further comprising:
a detection portion installed on a bottom surface of the elongated cavity and protruding toward the first surface; and
a driving portion configured to operate the second needle according to a signal detected by the detection portion,
wherein the detection portion is configured to signal the driving portion so that the second needle moves forward or backward in conjunction with an operation of the sliding portion.

8. The inhaler of claim 7, wherein the detection portion is configured to:
signal the driving portion so that the second needle moves forward when the sliding portion moves in a direction from the first surface toward the second surface; and
signal so that the second needle moves backward when the sliding portion moves in the longitudinal direction.

9. The inhaler of claim 6, wherein
a plurality of needle holes configured to penetrate the sliding portion in the longitudinal direction are formed in the sliding portion, and
the needle holes have a diameter of an opening adjacent to the second surface greater than a diameter of an opening adjacent to the first surface.

10. The inhaler of claim 6, further comprising:
a spring having one end coupled to the sliding portion and an other end coupled to a bottom surface of the elongated cavity,
wherein the spring is configured to contract when the sliding portion is pressurized and moves toward the second surface, and
wherein the spring is configured to support the sliding portion to be positioned spaced apart from the first needle and toward the first surface when the sliding portion is not pressurized.

11. The inhaler of claim 1, wherein the capsule contains a functional material in a powder form.
